# EUROPEAN PATENT APPLICATION

(11) **EP 4 289 458 A1**
(43) Date of publication of application: **13.12.2023**
(21) Application number: 23177944.8
(22) Date of filing: 07.06.2023
(51) Int. Cl.: A61M 5/36

(54) **AIR IN INFUSION LINE DETECTING DEVICE**

(30) Priority: 08.06.2022 EP 22177767
(71) Applicant: Micrel Medical Devices S.A., Konstantinoupoleos 42 Karela Industrial Area 19441 Koropi (GR)
(72) Inventor: Tsoukalis, Achilleas, 15669 Papagou (GR)
(74) Representative: Eisenführ Speiser

(57) **Abstract**

Disclosed is an air in infusion line detecting device, comprising an acoustic emitter (3) adapted to be provided at one side of an infusion line (5) and to vibrate at its resonance frequency so as to transmit an acoustic sound wave with a frequency corresponding to said resonance frequency, and an acoustic receiver (4) adapted to be provided at another side of the infusion line (5) and to be set into vibrations caused by the sound wave transmitted by said emitter (3) through the infusion line (5) and to generate an output signal indicating the characteristics of said vibrations. The device is characterized in that the resonance characteristics of said emitter (3) and/or the distance between said emitter (3) and said receiver (4) is adapted so that the sound wave is generated as a standing wave between said emitter (3) and said receiver (4).

## Description

### FIELD OF THE INVENTION

The present invention relates to a device which detects whether or not there is air in an infusion line as well as an infusion pump comprising such a device.

### BACKGROUND OF THE INVENTION

Air in line detectors or AIL detectors are electronic devices that can detect air bubbles in an infusion fluid path, wherein they are categorized in optic and ultrasound types. The present invention is about a detector of ultrasound category. Ultrasound has very good transmission characteristics generally in fluid and in particular in water and very poor transmission characteristics in air with Megahertz frequencies. Air or air bubbles in an infusion line for parenteral infusions may be fatal for the patient so that for an infusion it must be monitored whether air is in the infusion line. Preferably, an AIL detector comprises a vibrating piezoelectric plate as emitter provided at one side of an infusion line or tubing and another vibrating piezoelectric plate as receiver provided at the opposite side of the infusion line or tubing. If there are no air bubbles, sound is transmitted from the emitter to the receiver through the infusion line and, hence, through the infusion fluid, but when an air bubble is in the middle of the infusion line, sound is not transmitted, and an alarm is triggering a calculation algorithm to decide if an alarm will be emitted or not.

E.g. from US 5,053,747 A the use of frequency sweeps or scans is known to detect whether or not air is in the infusion line. US 8,539,812 B2 and US 8,739,601 B2 disclose to use a variable frequency signal for a frequency sweep or scan and to continue the frequency sweep or scan with the best frequency found while verifying with a second frequency, wherein the time of loss of the signal determines the size of the air bubble. According to US 9,498,583 B2 a threshold value is set and signals exceeding this threshold are detected while an alarm is set if a certain volume of air detected has passed through at some window time.

However, problems in an AIL detection result from false alarms. Further, for ambulatory infusion pumps low power is also very important.

It is an object of the present invention to provide an improved air in infusion line detecting device having a better reliability and requiring low power.

### SUMMERY OF THE INVENTION

In order to achieve the above and further objects, according to a first preferred aspect of the present invention, there is provided an air in infusion line detecting device, comprising an acoustic emitter adapted to be provided at one side of an infusion line and to vibrate at its resonance frequency so as to transmit an acoustic sound wave with a frequency corresponding to said resonance frequency, and an acoustic receiver adapted to be provided at another side of the infusion line and to be set into vibrations caused by the sound wave transmitted by said emitter through the infusion line and to generate an output signal indicating the characteristics of said vibrations, characterized in that the resonance characteristics of said emitter and/or the distance between said emitter and said receiver is adapted so that the sound wave is generated as a standing wave between said emitter and said receiver.

According to the present invention, the resonance frequency of the fluid channel or gap between the two acoustic elements is used, in order to determine the frequency to operate, wherein it is not arbitrary. This resonance happens when the emitting sound signal has a wavelength which is almost equal to or a pair fraction of the fluid gap. With this frequency standing waves and a resonance occur, resulting in the generation of the same vibration and the addition of new energy to the older decaying one. By doing this, according to the present invention it is used the resonance frequency of the piezoelectric element emitting most of the energy at a frequency f to a gap that has length being preferably the same as or a pair fraction (/2, 14, /8 etc.) of the emitted sound wavelength, so as to provide a resonance emitter and a sound resonance fluid transmission path with a standing wave resulting in the phenomenon that the energy at the receiver is higher than the energy of the transmitter due to the addition of energies of the past and new waves.

The emitter pushes liquid or air in front of it (through the tubing wall) like a piston transversely to the direction of flow of the fluid, and a wave is generated when the comeback of the "piston" happens before the front reaches the other side of the infusion line where the receiver is located. The wavelength λ of the ultrasound frequency f in water is longer than the wavelength in air, according to the formula: λ = v / f where v is the transmission speed in the medium v = 1.481 m/s in water and v = 343 m/s in air. So, if the system is made to resonate with standing waves in water where approximately λ is the water gap, the transmission is not possible with air through the gap between the two acoustic elements because of the acoustic reflection coefficient at a frequency of Megahertz.

The use of a frequency being the resonance frequency for the emitter, the receiver and the medium filled with water results in a better energy transmission and signal clarity at the reception so that the total emitting power can be reduced.

The ultrasonic coupling is achieved by the implementation of predetermined dimensions of the gap between the emitter and the receiver. Preferably, a position holder part of an infusion line lid or a pump cover is prevents the infusion line or tubing to accidentally go out of the predetermined correct coupling position, so that false alarms are avoided, since production tolerances can end up to some infusion pumps giving more often false alarms. Indeed, each of the two acoustic elements has a resonance frequency different than the nominal resonance frequency, and the fluid channel and the tubing segment arranged therein have also manufacturing dimensions that are not exactly nominal. So, in practice, when applying a frequency sweep, there is a frequency wherein all three parts are somehow out of the center of their Gauss curve but have an acceptable transmission and detection result. With the present invention, the adaption of the fluid channel to the standing wave dimension results in a much more power transmission so that even out of the center of the Gauss curve the signal is clear and no pump in the production will give any false alarms.

Preferred embodiments and modifications of the present invention are defined in the dependent claims.

Preferably, the resonance frequencies of both the emitter and the receiver are essentially identical.

According to a preferred embodiment, there is provided a processing unit which is adapted to control said emitter and to evaluate and/or process the output signal from said receiver.

According to a modification of the aforementioned embodiment, said processing unit comprises a frequency control which is adapted to control said emitter with respect to the frequency of the sound wave to be generated by said emitter.

According to a further modification of the aforementioned embodiment, said frequency control is adapted to control said emitter so as to carry out a frequency sweep or scan within a frequency range including inter alia the resonance frequency for detection of air in order to achieve an essentially optimal transmission of the sound wave in dependence on the distance between said emitter and said receiver and/or the characteristics of said emitter and/or said receiver due to an evaluation of the output signal carried out by said processing unit. The acoustic elements emit most energy at their resonance frequency. Dimensions and temperature vary this frequency from device to device, and so the prior art detectors use a frequency sweep to find a resonance frequency matching both emitter and receiver so that the receiver resonates, too, and output a high signal.

The transmission speed is affected by the temperature so that a sweep in frequencies guarantees the resonance and the transmission in water as well as the temperature despite of small mechanical structural variations. At the receiver side, the same acoustic element is used which also resonates with the frequency received wherein its signal strength and frequency are closer to its own proper resonance frequency. According to the present invention, since there is a standing wave in the fluid gap, the emitter and the receiver are in phase with each other, and this increases an acoustic pressure as both the emitter and the receiver oscillate at same time towards the fluid and away from it, so that in total less power for the emitter is needed compared to the prior art devices. So, the accuracy in detection is improved over the prior art.

The sweep frequencies are used around the chosen emission frequency. Since the AIL detector is not a general purpose detector, but a detector provided for a specific infusion pump, a mechanical dimension setup and tubing type and dimensions, the sweep of frequencies is around the combined same resonance frequency for water that has been determined mathematically and statistically in laboratory measurements for extreme tolerances of the construction and the temperature range. With the determination of the wavelength of the standing wave , also considered are the tubing walls which also transmit the sound. Being solids, the tubing walls result in a long wavelength and only at a small part affect the frequency to be used, so that the system frequency can be calculated or experimentally found in the laboratory and is very close to the fluid gap length between the internal walls of the tubing.

According to a further modification of the aforementioned embodiment, said frequency control is adapted to control said emitter so as to carry out the frequency sweep or scan around the resonance frequency within a range which at the start of the frequency sweep or scan is higher than a predetermined value and is reduced to said predetermined value during the continued frequency sweep or scan in order to decrease the power for the emitter and is extended beyond said predetermined value again in case said processing unit determines the absence of the output signal from the receiver in order to make sure that there is no error which might cause the absence of the output signal.

According to a further modification of the aforementioned embodiment, said frequency control is adapted to control said emitter so as to stop the frequency sweep or scan once said processing unit evaluates the output signal that said receiver receives an essentially clear sound wave.

According to a further modification of the aforementioned embodiment, said processing unit is adapted to determine from the output signal of said receiver during the frequency sweep or scan at least some frequencies of sound waves received by said receiver, and said frequency control is further adapted to further control said emitter so as to continue the frequency sweep or scan around these determined frequencies and, in case said processing unit determines the absence of the output signal, to repeat the frequency sweep or scan in essentially the same way in order to verify that it is air and no error which causes the absence of the output signal.

According to a further modification of the aforementioned embodiment, the frequency control is adapted to control the emitter so that a repetition rate of the frequency sweeps or scans is higher than a predetermined value in case said processing unit determines the absence of the output signal, and is reduced to said predetermined value so that said processing unit is able to determine the volume of air passed, wherein preferably said frequency control is adapted in such case to adjust the repetition rate so as to enable said receiver to be set into vibrations for detecting at least essentially all the air bubbles at the highest infusion rate used or to adjust it, preferably in a proportional manner, to the infusion rate in order to reduce power. In particular, when an air bubble is detected, the AIL test repetition rate can be increased in order to determine the exact time duration of the air detected at a known infusion rate and multiplied by the infusion rate so as to determine the air volume passed. This allows a lower power consumption if no air bubbles are detected and a better resolution if a bubble is detected.

According to a further modification of the aforementioned embodiment, said processing unit is further adapted in case of the presence of the output signal from said receiver to time stamp it, to calculate the air volume from the infusion rate by multiplying the infusion rate with the sweep or scan repetition period and to add in a shifting time window to other volumes already calculated as well as to signal an alarm in case a predetermined volume of air per time is exceeded, wherein in particular said processing unit can be further adapted to signal an alarm which is a locally visual and/or acoustical and/or vibrating signal, and preferably to transmit said alarm to a hospital or a cloud based server and then preferably to a hospital alarm system. So, the processing unit receives an air bubble presence signal (no signal of the receiver in a frequency scan), time stamps it as a bubble for the whole time until the next scan, then counts the volume of air passed through with the actual infusion rate and then, depending on pump settings, if a safety volume for humans is exceeded, gives an alarm. Whereas small air bubbles are passing as they do not result in a health thread, accumulated air bubbles may represent a threshold volume so that an alarm has to be given before the bubbles reach the tubing output to the patient. With a different setting, the volume of air is counted in a shifting time window (minute or hour), and an alarm is given if a certain air volume exceeds a predetermined limit for that window time.

Further, the processing unit can add the volumes of air bubbles found and does not give an alarm until said volume reaches a predetermined safety limit. Indeed, small air bubbles do not hurt the patients, since they are absorbed in the bloodstream. However, in case their total volume expand to usually a milliliter range, they may cause clots of blood (embolism) and endanger the patient, wherein the processing unit gives an alarm locally and, if it is a connected device, transmits said alarm to a server or a hospital alarm system. This is done before the air bubble reaches the end of the tubing of a generally long infusion line, so that it can be eliminated by a nurse or the patient himself if trained in homecare. The volume of the air bubbles which triggers an alarm may be programmed as a volume per detection or per time, which can be done e.g. in the pump. This depends on the age of the patient, and the connected pump having access to the EMR of the patient and his age may adjust this volume per hour automatically. By doing so, a shifting window of 60 minutes during which all air bubble sizes detected in the time window are accumulated is refreshed every time a new air bubble is detected so that an alarm is given in case the alarm limit is reached.

According to a further preferred embodiment, said processing unit further comprises at least one noise cancelling filter adapted to filter the output signal from said receiver.

According to a modification of the aforementioned embodiment, said at least one noise cancelling filter is a low pass filter or a band pass filter.

According to a further modification of the aforementioned embodiment, said processing unit further comprises a digitizer adapted to digitize the output signal from said receiver after having been filtered by said noise cancelling filter and to further filter the then digitized output signal wherein preferably said processing unit is further adapted to digitize the output signal only in case it is noiseless and to determine a precise band or even only one sweep or scan frequency period.

Accordingly, the low pass or bandpass filter is provided at the receiver output so as to eliminate RF and other parasite signals, and preferably then the signals are digitized with a comparator which sets a threshold voltage below which any signal is noise. In the digital domain, the provision of another filter is possible, that accepts only signal periods equal to those of the frequency emitted with the result in a further elimination of noise that may give signal while air is in the channel. In case during a frequency sweep, the receiver output a signal exceeding the threshold, the controller stops the scan to reduce power, and it is considered that air bubble or foam is not present until the next scan. All digital processing and digitization can be done by a processor as today's processors have the speed and peripherals to easily carry out such signal processing and filtering.

According to a further preferred embodiment, said processing unit is further adapted to determine whether or not the filtered output signal occurs concurrently with a frequency sweep or scan resulting in no detection of air, and said frequency control is further adapted to control said emitter in the absence of the output signal from said receiver to extend the frequency sweep or scan to all available frequencies and, if even then said processing unit determines the absence of the output signal from said receiver indicating that air is in the infusion line, to reduce the sweep or scan repetition period.

According to a further preferred embodiment, there is provided a single acoustic element which includes a combination of said emitter and said receiver and is adapted to be provided at one side of an infusion line, and further comprising a solid state wall element, preferably comprising hard plastic, adapted to be provided at the opposite side so as to reflect the sound wave transmitted by said emitter back to said receiver. Because of the generation of a high signal and a resonance that can last for some milliseconds, only one combined ultrasound element can be provided at one side of the infusion line and a hard passive plastic wall at the opposite side of the infusion line, wherein said combined ultrasound element can first emit the sound waves and then sense them after having been reflected from the opposite side at resonance frequency in case of the absence of air bubbles or not in case of the presence of bubbles.

Preferably, the emitter and/or the receiver comprises an ultrasound piezoelectric element.

According to a further preferred embodiment, there is provided a support including a cavity which is provided to accommodate the infusion line and a lid adapted to close said cavity and to engage the infusion line so as to be held it inside the cavity in a predetermined correct down position, wherein said emitter and said receiver are provided at said support.

According to a further aspect, the air bubble detection is provided as part of an infusion pump comprising an infusion mechanism, an infusion set part to be inserted into the AIL detector, a controller, a memory and a battery, as well as the air in infusion line detecting device of the first aspect with the use of algorithms to set AIL alarms and thresholds, and an alarm system generating local sound and visual alarms and preferably also wireless alarms to a distant server or cloud-based monitoring system or hospital alarm system.

The air in infusion line detecting device according to the first aspect of the present invention use a pair of piezoelectric plates positioned one opposite the other at a calculated gap for a specific infusion tubing segment that preferably is designed for the purpose. Especially, the infusion set comprises a silicone injection infusion segment that may have two polished flat sides at the upstream side and a calculated gap between the two flat sides. Since silicone injection tubing segments are formed by a mold, the mold may have polished or not polished surfaces and round or flat portions with a narrow space calculated for best detection flow path, wherein the polished and flat parallel surfaces are best for signal transmission. At the downstream side, the same AIL detecting device may be located after an air eliminating filter, so as to detect air in case the filter is damaged, and not to alarm in case the filter is functioning and eliminating air bubbles. In such a case, alarm is set only if lots of air are present as a possible result of an empty reservoir or an upstream leakage. Such a dual AIL infusion pump may give, safely for the user, much less false alarms than in the prior art when pumps with an air eliminating filter give alarms as the upstream AIL detector senses air bubbles that will be eliminated anyway by the said filter, which is annoying, especially with the parenteral nutrition when a patient receives nutrition at night while sleeping.

According to a further preferred embodiment, a short or larger range of frequencies is used for every air bubble detection scan with a specific short detection duration and a specific repetition rate (detections per second) for low power consumption. This repetition rate may be constant or variable, depending on the infusion rate, since air bubbles are passing through the tubing with a speed depending on the infusion rate (ml/h) and the cross section area at the detection point where the volume of air passed per time unit is determined. The repetition rate is calculated so as to detect any bubble passing, more frequent at higher infusion rates (ml/h) so that no bubble passing is lost or ignored. The infusion tubing between two ultrasound plates is somehow compressed so as to form a narrow fluid path and have at both sides a sufficiently flat surface so that sound can pass through. This also helps to have a complete absence of fluid when an air bubble passes through as the cross section is small and the speed locally is higher than in the rest of the infusion line, wherein the volume of air passed per time corresponds to the infusion rate.

According to a second aspect of the present invention, the above object is achieved by the provision of an infusion pump comprising the device according to the aforementioned first aspect of the present invention.

The aforementioned and other advantages of the present invention will become apparent from the following more detailed description when taken in conjunction with the accompanying drawings of illustrative embodiments.

### BRIEF DESCRIPTION OF THE DRAWINGS

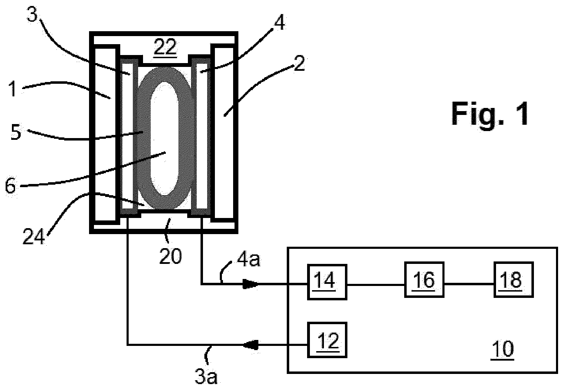

- Fig. 1: is a schematical cross-sectional view of an air in infusion line detecting device according to a first preferred embodiment of the present invention showing a housing, a piezoelectric emitter arranged at the one side of the housing parts, a piezoelectric receiver arranged at the other side of the housing and an infusion tubing arranged between the emitter and the receiver as well as a processing unit connected to the emitter and the receiver;

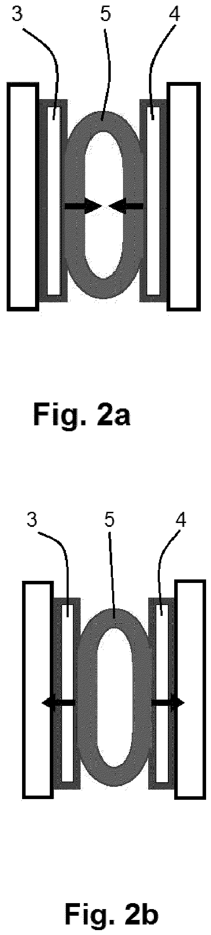

- Fig. 2a: schematically shows the embodiment of Fig. 1 (showing only parts of the housing and without showing the processing unit) in a vibrating moment wherein the inner surface of both the emitter and the receiver is in a maximum deflected position towards the tubing by the effect of a standing wave at a specific resonance frequency which is only indicated by arrows pointed to each other;
- Fig. 2b: schematically shows the embodiment of Fig. 1 (showing only parts of the housing and without showing the processing unit) in a vibrating moment wherein the inner surface of both the emitter and the receiver is in a maximum deflected position away from the tubing by the effect of the standing wave which is only indicated by arrows pointed away from each other; and

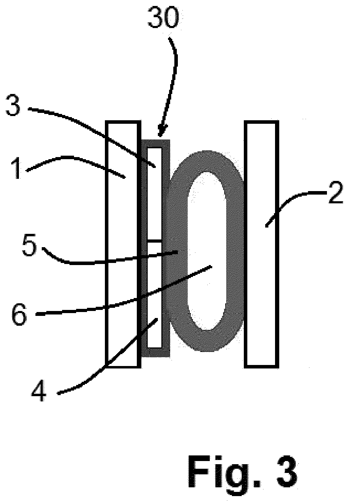

- Fig. 3: is a schematic cross-sectional view of an in infusion line detecting device according to a second preferred embodiment of the present invention showing only parts of the housing, a single acoustic element including a combination of the emitter and receiver and an infusion tubing arranged between the single acoustic element and the opposite part of the housing, without showing the processing unit.

### DESCRIPTION OF PREFERRED EMBODIMENTS

Fig. 1 shows an air in infusion line detecting device according to a first preferred embodiment comprising a housing of which a first plate 1 and a second plate 2 are visible and form two sidewalls of the housing spaced from each other wherein preferably said plates 1 and 2 can be made of hard plastic. An emitter 3 which preferably can comprise a piezoelectric plate is fixed with its outer surface to the first plate 1, in particular by bonding, and a receiver 4 which preferably can comprise a piezoelectric plate is fixed with its outer surface to the second plate 2, in particular by bonding. Further shown is an infusion line or tubing 5 which is provided in a sandwich arrangement between the emitter 3 and the receiver 4 so that the emitter 3 and the receiver 4 are arranged opposite to each other with respect to the infusion line or tubing 5 within which an infusion liquid 6 is flowing. In the shown embodiment the inner surface of both the emitter 3 and the receiver 4 is in contact with the outer surface of the infusion tubing 5 whereby the infusion tubing 5 is slightly compressed so to ensure a permanent contact with the emitter 3 and the receiver 4.

Fig. 2a schematically shows by representation of two arrows pointed to each other that, by the effect of a standing wave at a specific resonance frequency resulting in vibrations or oscillations of the emitter 3 and the receiver 4, the inner surfaces of both the emitter 3 and the receiver 4 move simultaneously towards the infusion tubing 5 in a first half of each oscillation cycle. And Fig. 2b schematically shows by representation of two arrows pointed away from each other that, by the effect of the standing wave, the inner surfaces of both the emitter 3 and the receiver 4 move simultaneously away from the infusion tubing 5 in a second half of each oscillation cycle. With respect thereto it is noted that the amplitude of the vibrations or oscillations is rather small and not visible.

Piezoelectric elements, when they vibrate, become alternatively thinner or thicker by the effect of electric power supplied to the piezoelectric emitting element or by the effect of acoustic sounds exciting the piezoelectric receiving element. In order to utilize this effect, according to a preferred embodiment both the emitter 3 and the receiver 4 comprise a piezoelectric plate or are provided as a piezoelectric plate.

As the back or outer surface of both the emitter 3 and the receiver 4 is fixed to the housing, their free side or inner surface acts like a reciprocally moving piston to generate acoustic waves or to react to the acoustic waves. In the shown embodiment the free side or inner surface of both the emitter 3 and the receiver 4 is in contact with infusion tubing 5 carrying the infusion liquid 6 that behaves acoustically like water. The emitter 3 resonates with a specific frequency so as to generate and emit acoustic or sound waves, and the receiver 4 also resonates as reaction to said acoustic waves and accordingly generates an output signal, wherein the receiver 4 has essentially the same frequency characteristics as the emitter 3. But it is the size of the gap which defines a path of the acoustic waves through the infusion fluid 6 and makes standing waves possible at that specific resonance frequency. These standing waves increase the power at the receiver 4 and hence its voltage. There are differences in resonance frequency even in piezoelectric plates of the same dimensions, so that for both the emitter 3 and the receiver 4 preferably plates may be selected as the same resonance pairs. Small differences between resonance frequencies of several pairs is compensated by carrying out a frequency sweep or scan that passes through all these frequencies. But since it is not possible to also 'sweep' the gap size, the standing waves cannot be exact but in a vicinity or an adjacent range with somehow lower than the maximum voltage in the receiver. As further shown in the figures, the tubing 5 is slightly compressed between the emitter 3 and the receiver 4 resulting in a smaller fluid width for two reasons, first to make a good contact with the emitter 3 and the receiver 4 at each side and second to reduce the fluid gap so to detect smaller air bubbles.

In presence of air in the tubing 5, the transmission is disturbed or interrupted so that the receiver 4 generates a small output signal, e.g. with a low or very low intensity, only or no output signal at all.

As further shown in Fig. 1, a processing unit 10 is provided which is schematically depicted as a block diagram. The processing unit 10 is adapted to control the emitter 3 through a connection line 3a and to evaluate and process the output signal from the receiver 4 through the connection line 4a. As also schematically shown in Fig. 1, the processing unit 10 comprises a frequency control 12, a noise cancelling filter unit 14, a digitizer 16 and an evaluation unit 18. The frequency control 12 is adapted to control the emitter 3 with respect to the frequency of the sound wave to be generated by the emitter 3. In particular, the frequency control 12 is adapted to control the emitter 3 so as to carry out a frequency sweep or scan within a frequency range which is assumed to also include the resonance frequency for detection of air in the infusion line 5 in order to achieve an essentially optimal transmission of the sound wave in dependence on the distance between the emitter 3 and the receiver 4 and/or the characteristics of the emitter 3 and/or the receiver 4 due to an evaluation of the output signal from the receiver 4 wherein such evaluation is carried out by the evaluation unit 18 of the processing unit 10. The range of the frequency sweep or scan around the resonance frequency may be larger at the start of the frequency sweep or scan and narrower during the continued frequency sweep or scan in order to decrease the power for the emitter 3 and is extended again in case the evaluation unit 18 determines the absence of the output signal from the receiver 4 in order to make sure that there is no error or variation which might cause the absence of the output signal. Further, the frequency control 12 controls the emitter 3 so as to stop the frequency sweep or scan once the evaluation unit 18 evaluates the output signal that the receiver 4 receives an essentially clear sound wave.

The evaluation unit 18 determines from the output signal of the receiver 4 during the frequency sweep or scan at least some frequencies of sound waves received by the receiver 4, wherein the frequency control 12 controls the emitter 3 so as to continue the frequency sweep or scan around these determined frequencies and, in case the evaluation unit 18 determines the absence of the output signal from the receiver 4, to repeat the frequency sweep or scan in essentially the same way, i.e. at the same repetition cycle, in order to verify that it is air and no error or variation, like e.g. a variation of the temperature, which causes the absence of the output signal from the receiver 4.

Further, the frequency control 12 controls the emitter 3 so that a repetition rate of the frequency sweeps or scans may vary and be higher than a predetermined value in case the evaluation unit 18 determines the absence of the output signal from the receiver 4, whereas the repetition rate is reduced to said predetermined value so that the evaluation unit 18 more precisely determines the length and, hence, the volume of air passed through the infusion line 5 between the emitter 3 and the receiver 4. In such case the frequency control 12 adjusts the repetition rate so as to enable the receiver 4 to be set into such vibrations which enable the detection of essentially all the air bubbles at the highest infusion rate in order to avoid any lost of air bubbles, or adjusts it in a proportional manner to the infusion rate in order to reduce power needed for the emitter 3. In case the evaluation unit 18 determines the presence of the output signal from the receiver 4, in the processing unit 10 the output signal is time stamped, the air volume is calculated from the infusion rate by multiplying the infusion rate with the sweep or scan repetition period and added in a shifting time window to other volumes which has already been calculated, wherein the processing unit 10 gives an alarm in case a predetermined or programmed volume of air per time is exceeded. Said alarm is a locally visual and/or acoustical and/or vibrating alarm and is preferably transmitted to a hospital or cloud based server and then preferably to a hospital alarm system.

As further schematically shown in Fig. 1, the noise canceling filter unit 14 filters the output signal from the receiver 4. The noise canceling filter unit 14 comprises one filter or a plurality of filters which may be a low-pass filter or a band-pass filter.

As also schematically shown in Fig. 1, the digitizer 16 is connected to the noise canceling filter unit 14 and digitizes the output signal from the receiver 4 after having been filtered by the noise canceling filter unit 14. The digitizer 16 may additionally filter the then digitized output signal in the digital domain but accepts only noiseless signals wherein a more precise band or even only one sweep or scan frequency period can be determined. Moreover, the evaluation unit 18 determines whether or not the filtered output signal occurs concurrently with a frequency sweep or scan which indicates a no air detection status, and the frequency control 12 controls the emitter 3 in the absence of the output signal from the receiver 4 as determined by the evaluation unit 18 so as to extend the frequency sweep or scan to all available frequencies and, if even then the evaluation unit 18 of the processing unit 10 determines the absence of the output signal from the receiver 4 indicating that air is in the infusion line 5, to reduce the sweep or scan repetition period.

As it is further retrievable from the Fig. 1, as parts of the housing shown are not only the plates 1 and 2, but also a bottom 20 and a lid 22, so that both the plates 1 and 2, the bottom 20 and the lid 22 encloses a cavity 24 which accommodates the infusion tubing 5. In the shown embodiment, the lid 22 closes the cavity 24 from the above and in its closed position engages the infusion tubing 5 so that it is held inside the cavity 24 in a predetermined correct down position against the bottom 20. So, the housing is provided as a support so as to hold the infusion tubing 5 inside the cavity 24 in the predetermined correct position.

Fig. 3 shows an alternative embodiment of the air in infusion line detecting device which comprises a single acoustic element 30 including a combination of the emitter 3 and receiver or at least an emitting and receiving function. This single acoustic element 30 is arranged at one side of the infusion tubing 5. At the opposite side of the infusion tubing 5 it is provided the second plate 2 as a solid state wall element, preferably comprising hard plastic, wherein this solid state wall reflects the sound wave transmitted by the emitter or emitting function back to the receiver or receiving function of the single acoustic element 30.

## Claims

1. An air in infusion line detecting device, comprising
an acoustic emitter (3) adapted to be provided at one side of an infusion line (5) and to vibrate at its resonance frequency so as to transmit an acoustic sound wave with a frequency corresponding to said resonance frequency, and
an acoustic receiver (4) adapted to be provided at another side of the infusion line (5) and to be set into vibrations caused by the sound wave transmitted by said emitter (3) through the infusion line (5) and to generate an output signal indicating the characteristics of said vibrations,
**characterized in that** the resonance characteristics of said emitter (3) and/or the distance between said emitter (3) and said receiver (4) is adapted so that the sound wave is generated as a standing wave between said emitter (3) and said receiver (4).

2. The device according to claim 1, wherein the resonance frequencies of both the emitter (3) and the receiver (4) are essentially identical.

3. The device according to claim 1 or 2, further comprising a processing unit (10) which is adapted to control said emitter (3) and to evaluate and/or process the output signal from said receiver (4).

4. The device according to claim 3, wherein said processing unit (10) comprises a frequency control (12) which is adapted to control said emitter (3) with respect to the frequency of the sound wave to be generated by said emitter (3).

5. The device according to claim 4, wherein said frequency control (12) is adapted to control said emitter (3) so as to carry out a frequency sweep or scan within a frequency range including inter alia the resonance frequency for detection of air in order to achieve an essentially optimal transmission of the sound wave in dependence on the distance between said emitter (3) and said receiver (4) and/or the characteristics of said emitter (3) and/or said receiver (4) due to an evaluation of the output signal carried out by said processing unit (10).

6. The device according to claim 5, wherein said frequency control (12) is adapted to control said emitter (3) so as to carry out the frequency sweep or scan around the resonance frequency within a range which at the start of the frequency sweep or scan is higher than a predetermined value and is reduced to said predetermined value during the continued frequency sweep or scan in order to decrease the power for the emitter (3) and is extended beyond said predetermined value again in case said processing unit (10) determines the absence of the output signal from the receiver (4) in order to make sure that there is no error which might cause the absence of the output signal.

7. The device according to claim 5 or 6, wherein said frequency control (12) is adapted to control said emitter (3) so as to stop the frequency sweep or scan once said processing unit (10) evaluates the output signal that said receiver (4) receives an essentially clear sound wave.

8. The device according to at least any one of the claims 5 to 7, wherein said processing unit (10) is adapted to determine from the output signal of said receiver (4) during the frequency sweep or scan at least some frequencies of sound waves received by said receiver (4), and said frequency control (12) is further adapted to further control said emitter (3) so as to continue the frequency sweep or scan around these determined frequencies and, in case said processing unit (10) determines the absence of the output signal, to repeat the frequency sweep or scan in essentially the same way in order to verify that it is air and no error which causes the absence of the output signal.

9. The device according to at least any one of the claims 5 to 8, wherein the frequency control (12) is adapted to control the emitter (3) so that a repetition rate of the frequency sweeps or scans is higher than a predetermined value in case said processing unit (10) determines the absence of the output signal, and is reduced to said predetermined value so that said processing unit (10) is able to determine the volume of air passed, wherein preferably said frequency control (12) is adapted in such case to adjust the repetition rate so as to enable said receiver (4) to be set into vibrations for detecting at least essentially all the air bubbles at the highest infusion rate used or to adjust it, preferably in a proportional manner, to the infusion rate in order to reduce power.

10. The device according to at least any one of the claims 5 to 9, wherein said processing unit (10) is further adapted in case of the presence of the output signal from said receiver (4) to time stamp it, to calculate the air volume from the infusion rate by multiplying the infusion rate with the sweep or scan repetition period and to add in a shifting time window to other volumes already calculated as well as to signal an alarm in case a predetermined volume of air per time is exceeded.

11. The device according to claim 10, said processing unit (10) is adapted to signal an alarm which is a locally visual and/or acoustical and/or vibrating signal, and preferably to transmit said alarm to a hospital or a cloud based server and then preferably to a hospital alarm system.

12. The device according to at least any one of the claims 3 to 11, wherein said processing unit (10) further comprises at least one noise cancelling filter (14) adapted to filter the output signal from said receiver (4).

13. The device according to claim 12, wherein said at least one noise cancelling filter (14) is a low pass filter or a band pass filter.

14. The device according to claim 12 or 13, wherein said processing unit (10) further comprises a digitizer (16) adapted to digitize the output signal from said receiver (4) after having been filtered by said noise cancelling filter (14) and to further filter the then digitized output signal wherein preferably said processing unit (10) is further adapted to digitize the output signal only in case it is noiseless and to determine a precise band or even only one sweep or scan frequency period.

15. The device according to claim 5 and according to at least any one of the claims 12 to 14, wherein said processing unit (10) is further adapted to determine whether or not the filtered output signal occurs concurrently with a frequency sweep or scan resulting in no detection of air, and said frequency control (12) is further adapted to control said emitter (3) in the absence of the output signal from said receiver (4) to extend the frequency sweep or scan to all available frequencies and, if even then said processing unit (10) determines the absence of the output signal from said receiver (4) indicating that air is in the infusion line (5), to reduce the sweep or scan repetition period.

16. The device according to at least any one of the preceding claims, comprising a single acoustic element (30) which includes a combination of said emitter (3) and said receiver (4) and is adapted to be provided at one side of an infusion line (5), and further comprising a solid state wall element (2), preferably comprising hard plastic, adapted to be provided at the opposite side so as to reflect the sound wave transmitted by said emitter (3) back to said receiver (4).

17. The device according to at least any one of the preceding claims, wherein the emitter (3) and/or the receiver (4) comprises an ultrasound piezoelectric element.

18. The device according to at least any one of the preceding claims, further comprising a support (1, 2, 20) including a cavity (24) which is provided to accommodate the infusion line (5) and a lid (22) adapted to close said cavity (20) and to engage the infusion line (5) so as to be held it inside the cavity (20) in a predetermined correct down position, wherein said emitter (3) and said receiver (4) are provided at said support.

19. An infusion pump comprising the device according to at least any one of the preceding claims.
